# EUROPEAN PATENT APPLICATION

(11) **EP 2 386 311 A1**
(43) Date of publication of application: **16.11.2011**
(21) Application number: 11174780.4
(22) Date of filing: 27.12.2002
(51) Int. Cl.: A61K 38/40, A61K 38/16, A61K 9/14, A61K 9/16, A61K 9/20, A61K 9/48, A61P 1/16, A61P 3/04, A61P 3/06, A61P 3/10, A61P 9/12, A23L 1/305, A61K 38/01

(54) **Compositions for improving lipid metabolism**

(30) Priority: 28.12.2001 JP 2001400641
(62) Divisional of application: 02793463.7
(71) Applicant: NRL Pharma, Inc., Kawasaki-shi, Kanagawa 213-0012 (JP)
(72) Inventor: Harada, Etsumori, Tottori-shi, Tottori 680-0941 (JP); Takeuchi, Takashi, Tottori-shi, Tottori 680-0945 (JP); Ando, Kunio, Kanagawa 213-0033 (JP); Shimizu, Hirohiko, Shizuoka 411-0803 (JP)
(74) Representative: Behnisch, Werner

(57) **Abstract**

The present invention provides a food additive or feed additive, comprising a composition containing lactoferrin as the active ingredient.

## Description

### TECHNICAL FIELD

The present invention relates to a pharmaceutical composition comprising lactoferrin and the like. The composition of the present invention can be used as an agent for improving lipid metabolism. More specifically, the composition of the present invention is useful for treating hypercholesterolemia, hyper-neutral lipidemia, hyper-low density lipoprotein (LDL) cholesterolemia, hypo-high density lipoprotein (HDL) cholesterolemia, obesity, fatty liver and cholesterol gallstone, and further for treating lifestyle-related diseases such as severe obesity, hyperlipidemia, hypertension and type II diabetes. The composition of the present invention can improve basal metabolic rate.

### BACKGROUND ART

Our time is an age of plentiful food. With it, obesity has surfaced as an important health problem which has to be overcome. In the United State of America, about 30% of elementary school children are regarded as overweight children who exceed the standard weight by 30% or more. As is clear from various epidemiological surveys, the obesity of school age mostly proceeds after attaining manhood. With it, overweight persons who exceed the standard weight by 30% or more appear in succession at the aging stage of from the second half of 30 years old to middle age, and thus the total obesity population in Western countries has accounted for a large ratio which human beings have never experienced. Although not so conspicuous as in Western countries, the increase of overweight children due to insufficient exercise and satiation has come to a serious problem even in our country.

Needless to say, obesity is an important dangerous factor to the onset of ischemic heart disease, essential hypertension, type II diabetes or a certain type of cancer, and the age of severe obesity is becoming lower and lower to announce a sudden increase of lifestyle-related diseases beforehand. Further, also fatty liver and cholesterol gallstone are diseases with obesity and lipid metabolic disorder for a background which cannot be overlooked. Obesity not only accelerates the onset of lifestyle-related diseases but also inactivate activities to psychologically making adaptability to the social life difficult. Accordingly, obesity is a large economical and social loss. In the countries where overweight persons are suddenly increasing, the development of methods of preventing/treating obesity while maintaining physical condition is a pressing need.

Above all, drugs or processed foods which can inhibit the absorption of fat in the digestive tract without side effects are essential in the prevention/treatment of obesity. The obesity due to overeating increases the cholesterol concentration present in blood low density lipoprotein (LDL) and conversely decreases the cholesterol concentration present in blood high density lipoprotein (HDL) to trigger arteriosclerosis. Accordingly, the development of drugs or processed foods which can increase blood high density lipoprotein (HDL) cholesterol and conversely can decrease low density lipoprotein (LDL) cholesterol is one of the most globally demanded developments. Since statin drugs such as provastatin, simbastatin and atorvastatin which inhibit hydroxymethylglutaryl conenzyme A reductase to reduce the synthetic amount of the internal cholesterol play an important role in treating the diseases of the circulatory system, the treatment of obesity, that is, lipid metabolic disorder is clearly a most urgent problem.

The cause of obesity is overeating. By controlling the appetite and increasing the physical energy consumption together with therapeutic exercise such as jogging, anyone may well reduce weight theoretically. However, to eat moderately and take exercise of its own volition is not so simple as in words, and even if it is possible to eat moderately, reality is not so simple. It is said that to change eating habits established in childhood is the most difficult habit among the many. The appetite is one of pleasures which can be easily satisfied. Furthermore, on becoming obese, thermal energy is reduced in consumption and stored as the body fat that much more, and thus obesity is caught in a vicious circle of bringing about obesity. The reduction in weight by moderation in eating brings forward a health problem as well. The reduction in weight by the reduction in energy intake not only reduces the white fat tissue whose decrease is desired but also reduces the weight of parenchymal organs at the same rate. The weight reduction in parenchymal organs lowers immunological competence, and the body resistance to pathogenic virus and pathogenic microorganisms is weakened to be readily seized with a disease such as a cold. Thus, what is demanded in this time of day is a weight reduction method of selectively burning the neutral fat stored in the white fat tissue without accompanying the weight reduction of parenchymal organs.

As the drugs which act on the central nerves to cause moderation in eating, the major ones are drugs such as madindol [C. Sirtori et al., Am. J. Med. Sci. 261:341-7(1971)] which act on the feeding center to reduce the appetite or drugs such as a stimulant and an amphetamine compound [O.J. Kalant, "The Amphetamines; Toxicity and Addiction" (Thomas, Springfield, 1966)] which exhibit strong dissimilation and feed glucose by decomposition of the body constituents to reduce the appetite. However, madindol has a side effect of inducing strong constipation to cause liver disorder. Further, the amphetamine drugs are habit-forming, and once fallen into drug dependence, it is very difficult to get out of it, and thus they have not been used as anoretics at all.

A therapeutic method of shortening the small intestine by excision by a surgical operation to reduce the area of the small intestinal mucosa which relates to digestion and absorption. However, for improvement of obesity or hyperlipidemia, excision of part of the sound digestive tract is not the proper way. There is a possibility that the danger of a pathogenic infection by the abdominal surgical operation or the influence by shortening of the small intestine will be surfaced as some disease with ages.

Western-type meals are different from meals of the East Asian countries including Japan which use carbohydrates as the major energy source. The ratio of neutral fat in caloric intake among the Western foods is estimated 35% to 43% although it slightly varies in every area. Ideally, the ratio of fat in energy intake is preferably 30% or less, and furthermore it is recommended that animal fat and butterfat which mostly contain saturated fatty acids are not more than half of the fat to be taken in and the remainder is taken in as vegetable oil and fish oil which largely contain unsaturated fatty acids. However, foods such as meat and dairy products which richly contain animal fat and butterfat are delicious and have high satisfaction of the taste, and thus it is next to impossible to reduce the intake of saturated fatty acids by refraining from taking in such foods. Anyhow, the Western-type meals use fat as the major energy source, and thus to inhibit the absorption of the neutral fat present in the foods taken in by the digestive tract is the most rational and effective preventing/treating method. That is why a limitation in the prevention/ treatment of obesity by moderation in eating and exercise exsists.

The dietary neutral fat and ester type cholesterol form fine micelles whose surface layer is covered with bile acid present in the bile, cholesterol and protein in the small intestine. Lipase to be secreted from the pancreas attaches to the micelle surface to decompose neutral fat into two fatty acids and monoglyceride which are absorbed from the mucosa of the small intestine. Thus, a drug binding to lipase in the cavity of the small intestine to inactive enzyme activity reduces the amount of neutral fat to be hydrolyzed, and resultingly inhibits the absorption of neutral fat by the digestive tract. On the other hand, a substance which lowers hydrophilicity acting on the interface of the micelle surface layer of neutral fat in contact with water, reduces the surface area on which lipase acts by the fusion of the micelles with each other, and accordingly the hydrolysis of neutral fat is decreased and as a result, the absorption of neutral fat by the digestive tract is inhibited. In other words, in order to inhibit the absorption of neutral fat taken in as meals by the digestive tract, there are two methods, that is, a method of administering a lipase inhibitor and a method of reducing the micelle surface layer of neutral fat on which lipase acts and decreasing the hydrolysis of neutral fat to reduce the absorption of neutral fat by the digestive tract.

As the former, orlistat [H.S. Fleury et al., Int. J. Obesity 11 (Suppl.3): 35-42 (1987)] is the only lipase inhibitor that is put to practice. Orlistat is widely used as a therapeutic drug for reducing the weight of a severely obese person, and it is reported that about 30% of the dietary fat taken in is inhibited from absorption and discharged. However, as the defects, there are frequent diarrhea, abdominal distension and gas generation, and furthermore it is reported that hypertension and liver failure are induced as the systemic side effect. Orlistat explosively came to wide use at the time of release but gradually ceased to be used due to a rash of side effects and at present, the amount of use is one third of its zenith. When orlistat is used for a long period of time in order to reduce weight, periodical monitoring is required and orlistat is mainly being used for severely obese persons under the supervision of a doctor.

Drugs or processed foods which reduce the area on which pancreatic lipase acts by fusing the micelles of dietary fat with each other and inhibit the absorption of dietary neutral fat have not been widely put into practice. However, purothionin (JP-A H04-300839) of a protein present in wheat malt, e-polylysine (JP-A H04-221320) of an antibiotic which is put into practice as an antiseptic for foods and a basic protein or a basic peptide such as protamine, histone and poly-L-arginine (JP-A H03-284627) inhibit the pancreatic lipase activity and exhibit the effect of inhibiting the absorption of fat in the digestive tract.

These methods by which the absorption of fat by the digestive tract may be inhibited cannot escape a defect included in themselves. The defect is that a large amount of the neutral fat which has escaped digestion and absorption in the small intestine flows into the large intestine. Various types of intestinal bacteria live in the digestive tract of mammalian species, and it is calculated that 150 trillion of more than 100 types of intestinal bacteria live in the large intestine. The intestinal bacteria living in the large intestine live on residual foods undigested in the digestive process in the small intestine or a very small amount of nutrients which have escaped digestion and absorption. In other words, the large intestine is an environment of poor nutrition supply, and bacteria appropriate for the environment of poor nutrition live. Furthermore, importantly, these intestinal bacteria are in a symbiotic relationship with the host, and produce lower fatty acids including lactic acid which are essential in the exhibition of the function of the large intestine to provide the host with them. However, when the large intestine whose absorption of fat is inhibited changes into a nourishing environment, it is impossible to remove a possibility that microorganisms appropriate for the environment of eutrophication explosively proliferate to overwhelm *Lactobacillus bifidus,* lactic acid bacteria and the like which are necessary to maintain health. The side effects such as diarrhea, gas generation and abdominal distension recognized in the administration of orlistat reflect the change of an intestinal bacterial plexus in the large intestine.

### DISCLOSURE OF THE INVENTION

The present inventors have found that when the lactoferrin to be obtained from cow milk is processed into enteric coated preparations of lactoferrin and orally administered to able-bodied persons and sick persons, the lipid metabolism can be quickly and significantly improved. Namely, on administering enteric preparations of lactoferrin to able-bodied persons and sick persons, the reduction in blood cholesterol level and blood neutral fat level, the rising in blood HDL cholesterol level and the reduction in blood LDL cholesterol level are caused with a statistically significant difference to accompany the improvement of the morbid condition such as essential hypertension and type II diabetes. Namely, clinical action to improve lipid metabolism is clear.

Further, it has been found that on investigations of the action to improve lipid metabolism with the use of rodents fed with a high fat feed, the cholesterol and the neutral fat in the liver are significantly reduced in addition to the same change of blood lipid as in humans. In other words, it has been found that lactoferrin inhibits the lipid accumulation in the liver by inhibiting the absorption of dietary cholesterol and neutral fat by the digestive tract and has the action to improve the profile of blood lipid.

It is confirmed (Kawase et al., "Dairy Science and Food Study", vol. 45, A75 to 81, 1996) that oral administration of latoferrin to humans has an effect of increasing *Lactobacillus bifidus.* Even on oral administration of lactoferrin to humans, side effects such as diarrhea, gas generation and a feeling of abdominal distension which are seen with substances for inhibiting fat absorption have hardly been recognized. In other words, even when the fat which has escaped the digestion and absorption in the small intestine flows into the large intestine, the lactoferrin group protein proliferates useful intestinal bacteria such as *Lactobacillus bifidus,* and can be said to have an advantage of hardly causing harmful effects with the alternation of intestinal bacteria. In view of this, it has been thought that the lactoferrin group protein can be continuously used for a long period of time.

On the other hand, since yolk contains a large amount of cholesterol, hen eggs are misunderstood to induce arteriosclerosis and are unpopular food in Western countries in spite of its high nutritive value. However, unexpectedly, hen eggs have hardly any action to raise blood cholesterol level. For example, the experimental result of allowing six able-bodied volunteers to take in ten boiled eggs a day for many days in succession is published, and a significant rising in blood cholesterol level did not occur even by taking in them for one week or more. Yolk contains a large amount of fats and oils and lecithin together with cholesterol, and the fats and oils are easily dispersed in water as fine micelles, and accordingly the absorption of cholesterol dissolved in the fats and oils by the digestive tract is expected to be quick. However, the reason why the blood cholesterol level does not rise even when a large amount of hen eggs are taken in over a long period of time is thought due to the possible presence of a substance which hinders the absorption of cholesterol by the digestive tract. The important matter is that even when a large amount of boiled eggs whose proteins are denatured, the inhibition of the rising in blood cholesterol level is recognized, and it was thought that a substance for inhibiting the absorption of cholesterol present in hen eggs is not affected by heating or active substance exists in the peptides to be formed by partial decomposition with digestive enzyme after denaturation although it might be a protein.

For example, the Poultry Association reported that the administration of egg white reduced the LDL cholesterol level in blood and raised the HDL cholesterol level in blood in the clinical test by adult able-bodied volunteers. Thus, it was thought that the substance to inhibit the rising in cholesterol level which existed in hen eggs existed in egg white. Nagaoka et al., Department of Agriculture of Gifu University reported that when egg white was fractionated by taking the reduction in blood cholesterol in rodents as an indicator, the substance which exhibited activity was ovomucin (Information and Research on Stock Raising, Report, August, 2000, Monthly Report, Domestic Edition). However, the ovomucin in the egg white solids is around 4%, and thus considerable cost is required for commercialization by extracting ovomucin from egg white. Further, there is no clinical test to examine the variation of blood lipid by oral administration of ovomucin, and accordingly it is unknown whether the ovomucin is effective for a human or not.

By taking a hint from the action of lactoferrin to improve lipid metabolism, the present inventors investigated the absorption of fat and the influence on blood lipid by fractionating egg white to orally administer each fraction to rodents. This is based on the reason that egg white contains conalbumin corresponding to the mammalian lactoferrin and its content is around 10% or less next to ovalbumin. It is clear that the conalubumin of avian species is a protein corresponding to the lactoferrin of mammalian species by the comparison of the structures elucidated by the X-ray diffraction of the crystals (H. Kurokawa et al., J. Biol. Chem., 274:28445-28453, 1999). As the result of experiments, the present inventors have found that the conalbumin of the lactoferrin of avian species inhibits the absorption of dietary fat as expected to improve the profile of blood lipid as with lactoferrin.

On the basis of such knowledge, the present inventors have completed a composition for improving lipid metabolism which has at least one kind to be selected from the group consisting of a lactoferrin group protein comprising lactoferrin and conalubumin and an enzymatically decomposed product of the lactoferrin group protein comprising peptides corresponding to lactoferricin and lactoferricin of conalbumin as an active ingredient and a composition for treating at least one disease or condition to be selected from the group consisting of hypercholesterolemia, hyper-neutral lipidemia, hyper-low density lipoprotein (LDL) cholesterolemia, hypo-high density lipoprotein (HDL) cholesterolemia, obesity, fatty liver and cholesterol gallstone which has at least one kind to be selected from the group consisting of a lactoferrin group protein comprising lactoferrin and conalubumin and an enzymatically decomposed product of the lactoferrin group protein comprising peptides corresponding to lactoferricin and lactoferricin of conalbumin as an active ingredient. The composition of the present invention is effective for treating lifestyle-related diseases such as severe obesity, hyperlipidemia, hypertension and type II diabetes.

### BRIEF DESCRIPTION OF THE DRAWINGS

Figure 1 represents graphs showing the rising in the protein concentration in blood by administration of lactoferrin and the reduction in neutral at level and free fatty acid level (Example 1). The latticed bars on the left side show a control group and the dotted line bars on the right side show a lactoferrin group. The bars show standard deviations (each n=8). **P<0.01, in Student's t-test
Figure 2 is a graph showing a change in mouse blood cholesterol by administration of lactoferrin (Example 1). The latticed bars on the left side show a control group and the dotted line bars on the right side show a lactoferrin group. The bars show standard deviations (each n=8).
   **P<0.01, in Student's t-test
Figure 3 shows a ratio of the HDL cholesterol level to the total cholesterol level in mouse blood (Example 1). The latticed bars on the left side show a control group and the dotted line bars on the right side show a lactoferrin group. The bars show standard deviations (each n=8). ** P<0.05, in Student's t-test
Figure 4 represents graphs showing a change in the lipid content in the liver by lactoferrin (Example 2). The latticed bars on the left side show a control group and the dotted line bars on the right side show a lactoferrin group. The bars show standard deviations (each n=8). *P<0.05 and **P<0.01, in Student's t-test
Figure 5 is a graph showing the effect of enteric coated tablets of lactoferrin on neutral fat level (Example 3). ● and ○ show the case of oral administration of lactoferrin enteric tablets and Ⓧ shows the case of no oral administration.
Figure 6 is a graph showing the effect of enteric coated tablets of lactoferrin on total cholesterol level (Example 4). P<0.01 in Student's paired t-test

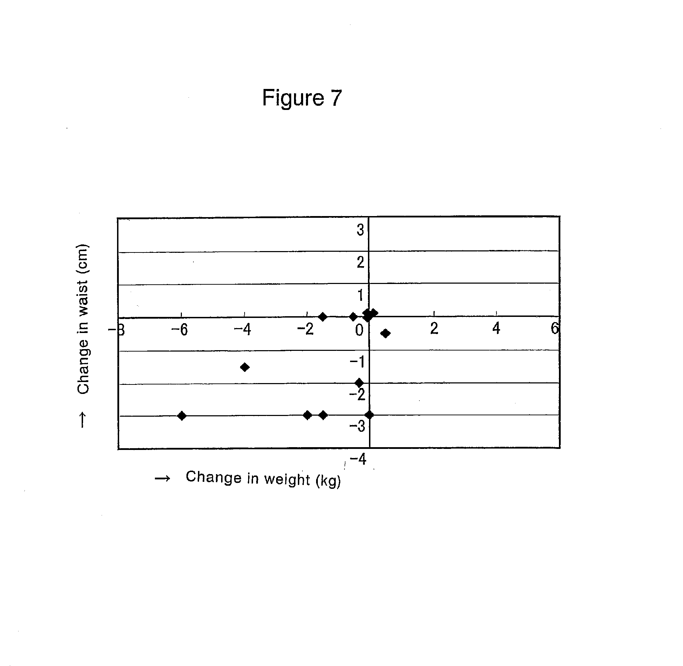

Figure 7 is a graph showing the effect of enteric coated tablets of lactoferrin on waist and body weight.
Figures 8 to 11 show the effect of enteric coated tablets of lactoferrin on neutral fat level and total cholesterol level (Examples 5 to 8).
Figure 12 is graph showing the effect of enteric coated tablets of lactoferrin on neutral fat level and total cholesterol level in detail (Example 9). ● shows a measured value of neutral fat the next morning after drinking.
Figure 13A is a graph showing a lactoferrin concentration in blood when lactoferrin enteric tablets were orally administered (Example 10).
Figure 13B is a diagram showing the schedule of administration of enteric coated tablets of lactoferrin and collection of blood in measuring the lactoferrin concentration in blood.
Figure 14A is a graph showing the result of measuring the body temperature at rising and the body temperature one hour after lunch with a group administered with lactoferrin and Figure 14B is a graph showing that result with a control group (Example 11).

### DETAILED DESCRIPTION OF THE INVENTION

The composition of the present invention has at least one of lactoferrin group proteins or enzymatically decomposed products of the lactoferrin group protein as an active ingredient. The "lactoferrin group protein" as used in the present specification includes lactoferrin and conalbumin, and the "enzymatically decomposed product of the lactoferrin group protein" includes a peptide corresponding to lactoffericin of the lactoferrin group protein.

For the composition of the present invention, any of the lactoferrin group protein and the enzymatically decomposed product of the lactoferrin group protein can be used as far as it exhibits the action to improve lipid metabolism and the action to basal metabolism rate on oral administration. Lactoferrin is a large molecule having a molecular weight of about 80,000 and has properties to form a chelate with two trivalent iron ions, and the "lactoferrin" as used in the present specification includes all types of lactoferrins ranging from the iron ion-free type to the type with iron ions completely saturated which can be human, bovine and recombinant lactoferrins independently of their origins.

The composition of the present invention may comprise only one type of lactoferrin and conalbumin or two types.

The composition of the present invention is to be orally administered. Its form may be a pharmaceutical composition and may be a food, a drink or a drinkable preparation. The composition of the present invention is preferably in a dosage form convenient for oral administration, for example, in the form of a dusting powder, a powder, a granule, a tablet or a capsule.

The fillers to be used in making the composition of the present invention into pharmaceutical formulations include, for example, monosaccharides and disaccharides such as lactose, sucrose, glucose, sorbitol and lactitol; starches such as corn starch and potato starch; crystalline cellulose; and inorganic substances such as light silica gel, synthetic aluminum silicate, calcium metasilicate aluminate and calcium hydrogenphosphate. Provided that there is a danger that the reducing monosaccharides and disaccharides cause aminocarbonylation reaction with the e-amino group of the lactoferrin group protein (or the enzymatically decomposed product of the lactoferrin group protein) as the active ingredient to denature it. Particularly, the presence of water and iron ions might advance quick aminocarbonyl-ation reaction.

The fillers to be used in making the composition of the present invention into pharmaceutical formulations include, for example, starches, carboxymethylcellulose (CMC), hydroxypropylcellulose (HPC),
carboxymethylcellulose sodium salt and
polyvinylpyrrolidone as the disintegrators. As the lubricants, sucrose fatty acid esters, calcium stearate and magnesium stearate can be used.

The composition of the present invention may be administered singly or used together with other drugs. Further, the composition of the present invention can be added to a food and a feed in administtation.

The composition of the present invention is preferably made into pharmaceutical preparations in a dry state. Lactoferrin (hereinafter lactoferrin is explained as the central figure but the same can be said when other active ingredients are used) of a representative active ingredient of the composition of the present invention is unstable at high temperature and high humidity. More specifically, the amino group of lactoferrin can cause amino-carbonyl reaction with a reducing group present in the fillers or the like. Through many stages, this reaction leads to the formation of a brown dye by irreversibly polymerizing the reaction products (browning reaction). The presence of a substance which catalyzes oxidation and high temperatures accelerate this reaction. Namely, in making lactoferrin into pharmaceutical preparations, if water is present, the amino-carbonyl reaction can be accelerated by the influence of Fe³⁺ or the like present in lactoferrin. Further, the heat generation to be caused by tableting further accelerates this reaction as well. Thus, in order to obtain stable lactoferrin pharmaceutical preparations which maintain the pharmacological effect, the pharmaceutical preparations should be made in a dry state as much as possible.

Since the lactoferrin powder as such cannot be tableted due to its very light specific gravity, in order to obtain the composition of more stable pharmaceutical preparations which maintain the pharmacological effect, for example, the active ingredient is mixed with a filler, a binder and a disintegrator, and the mixture is subjected to strong pressure molding by a slug machine to form a thin, large, flat disk and the disk is pulverized and put through a sieve to make the size of granules uniform. And, in preparing tablets, the granules are added with a lubricant and tableted and, if desired, coated with a coating film to make products. In case of capsules, a predetermined amount of granules is encapsulated to form capsules.

It is preferred that the composition of the present invention is made into enteric coated preparations. The present inventors hypothesize that a structure which may be called a lactoferrin sensor exists on the intestinal mucosa as descried in detail in International Application PCT/JP01/ 10212 (WO02/41912) and, on the other hand, have knowledge that lactoferrin is highly sensitive to pepsin but remarkably resistant to other proteases. Namely, for the lactoferrin having an acting site on the intestinal mucosa and, simultaneously, high sensitivity to pepsin, making it into enteric coated preparations particularly has a technical significance.

In order to make lactoferrin enteric, granules containing the active ingredient are filled into enteric capsules with a film having, as the major component, a base which has resistance to the gastric juice and dissolves in the small intestine, for example, a base to be selected from the group consisting of shellac, zein, hydroxypropylmethyl-cellulose phthalate, carboxymethylethylcellulose, cellulose acetate phthalate, a methacrylic acid copolymer, water-insoluble methylethylcellulose and an aminoalkyl methacrylate copolymer or a lubricant is added to granules containing the active ingredient to effect tableting and the obtained tablets may be coated with the film.

Particularly, the present inventors have confirmed lactoferrin in the blood of persons orally administered with enteric coated tablets of lactoferrin. Such knowledge could not have been obtained with the conventional tablets having lactoferrin as an active ingredient. The form of enteric coated preparations having lactoferrin as an active ingredient is one of preferred embodiments of the present invention. Furthermore, the form of enteric coated formulations which are made in the dry state and, simultaneously, have lactoferrin as an active ingredient is one of particularly preferred embodiments of the present invention.

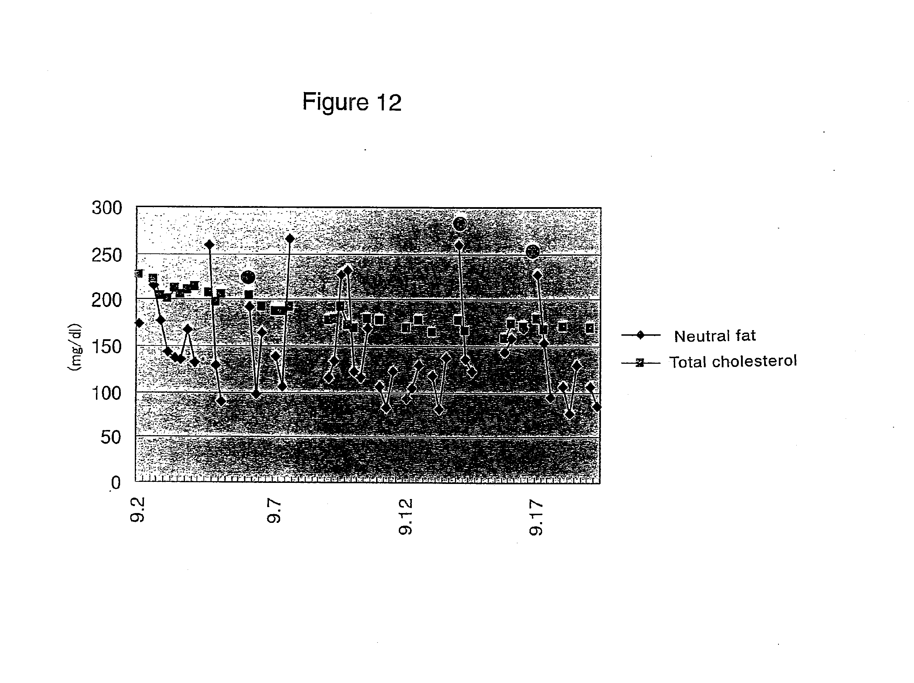

The administration of enteric coated tablets of lactoferrin and the collection of blood were performed according to the following schedule. Namely, after eating breakfast at 7:00, the blood before administration of lactoferrin was collected a little before 9:30 (Pre-sampling), and enteric coated tablets of lactoferrin (Preparation Example 5) were administered at 9:30, and then the blood was collected at 13:30 and 17:30 (4 hr-sampling and 8 hr-sampling, respectively) (Figure 12B).

Whether the prepared composition is enteric or not can be confirmed by testing its disintegrable properties with the use of a first solution (pH 1.2, General Testing Method 41 of the Pharmacopoea Japonica) obtained by adding 4 ml of diluted hydrochloric acid and water to 2.0 g of sodium chloride to dissolve it to form a solution of 1,000 ml and a second solution (pH 6.8) obtained by adding 118 ml of 0.2 N sodium hydroxide test solution and water to 250 ml of 0.2 M calcium dihydrogenophosphate to dissolve it to form a solution of 1,000 ml. When the tablets or granules which do not disintegrate on immersion in the first solution for 120 minutes but disintegrate on immersion in the second solution for 60 minutes do not dissolve in the stomach and start disintegration for the first time on flowing into the duodenum to elute the active ingredient, they can be judged enteric.

The composition of the present invention can exhibit an effect of improving the lipid profile in blood. On account of this, the composition of the present invention can be used in treating hypercholesterolemia, hyper-neutral lipidemia, hyper-low density lipoprotein (LDL) choleste-rolemia and hypo-high density lipoprotein (HDL) choleste-rolemia.

Further, the composition of the present invention can be also used in treating obesity, fatty liver and cholesterol gallstone.

Furthermore, with the composition of the present invention it can be thought that the active ingredient such as lactoferrin inhibits the intestinal absorption of dietary lipid to improve lipid metabolism and can reduce the energy intake to exhibit a weight reduction effect as well. Accordingly, the composition of the present invention is useful for treating lifestyle-related diseases such as severe obesity, hyperlipidemia, hypertension and type II diabetes.

In addition, the composition of the present invention can improve basal metabolic rate. For example, the composition raises the body temperature at rising and/or increases a difference between the body temperature at rising and the body temperature in action (for example, the body temperature several hours after rising and the body temperature one hour after meals). The basal metabolic rate means a minimum consumption of energy necessary for maintaining life in an awake state, and the basal metabolism starts decreasing with ages and decreases when vitamins and proteins are deficient and the temperature is high, and during sleeping as well. It is known that at the same sex and the same age, the basal metabolism is proportional to the body surface area. Accordingly, an obese person of the same weight with a higher amount of body fat and a smaller ratio of muscles generally has lower basal metabolism. Accordingly, an obese person is inferior in basal metabolic rate to a person who is not obese. The composition of the present invention can be used for improving the basal metabolism of an obese person and the like

The active ingredient of the present invention can proliferate *Lactobacillus bifidus* and lactic acid bacteria in the lower digestive tract, and accordingly can be administered without side effects of flatus, diarrhea, abdominal distension and the like.

The composition of the present invention is typically administered to a patient before meals, after meals, between meals or at bedtime in an amount of about 0.1 mg to about 50,000 mg, preferably about 0.5 mg to about 10,000 mg, more preferably about 10 mg to about 2,000 mg a day as the active ingredient at one time or dividedly. The dose can be individually determined in accordance with the age and weight of a patient to be administered and the object of administration.

The present invention will be concretely explained below by examples but the present invention is not to be limited to them.

### Preparation Example 1

One part of lactoferrin and one part of potato starch were thoroughly mixed, and compressed by a slug machine without using water into a disk, and the disk was pulverized to collect granules passed through a 16-mesh sieve, and filled in each hard capsule of No.1 of the Japanese Pharmacopoeia in an amount of 150 mg. Preparation Example 2

Five point five kilograms of lactoferrin, 8 kg of lactose, 10 kg of crystalline cellulose, 1 kg of carboxymethylcellulose calcium, and 0.5 kg of a glycerin fatty acid ester were thoroughly mixed and subjected to dry granulation in the same manner as in Example 1, and then the resulting granules were pressure-molded into tablets each tablet containing 50 mg of lactoferrin and having a diameter of 8 mm and a weight average of 250 mg.

### Preparation Example 3

Six kilograms of conalbumin, 10 kg of corn starch, 8.8 kg of hydroxypropylcellulose and 0.2 kg of magnesium stearate were mixed, and subjected to dry granulation in the same manner as in Example 1, and then the granules were pressure-molded into tablets each tablet containing 50 mg of conalbumin and having a diameter of 8 mm and a weight average of 250 mg

### Preparation Example 4

Ten kilograms of spray dried egg white powder (a content of conalbumin of 8.4%), 10 kg of potato starch, 0.5 kg of silica gel were mixed in a dry state, and pressure-molded in the same manner as in Example 2, and then the disk was pulverized to collect particles having a particle diameter of not greater than 0.1 mm which was then pressure-molded into a tablet having a diameter of 10 mm and a weight average of 800 mg.

### Preparation Example 5 (Preparation of Enteric Coated Tablet of Lactoferrin)

Five point five kilograms of lactoferrin, 8 kg of lactose, 10 kg of crystalline cellulose, 1 kg of carboxymethylcellulose calcium and 0.5 kg of a glycerin fatty acid ester were mixed and subjected to dry granulation in the same manner as in Example 1, and then the granules were pressure-molded into tablets each containing 50 mg of lactoferrin and having a diameter of 8 mm and a weight average of 250 mg. These tablets were placed in a coating machine (Hicoater HCT-48N, manufactured by Freund Industry Co., Ltd.), and sprayed with a fluid obtained by dissolving 30 parts of shellac and 7 parts of castor oil into 63 parts of isopropanol in a calculated amount to produce tablets provided with 10%, based on the weight of the tablets, of enteric coating.

### Example 1

Sixteen ICR line male mice of 5 weeks old were randomly classified into two groups of 8, and a control group was bred with a standard feed for rat and mouse (CE-2, a product of Japan Crea Co., Ltd.), and the other group was bred with CE-2 added with 1% lactoferrin (a product of Tatua Milk Biologix, in New Zealand, purity 84%) for four weeks. During this time, body weight was measured every three days, and with the lactoferrin group the body weight increased at a slightly quicker rate compared to the control group but there was no significant difference between both groups. Further, there was no significant difference in the weight of the liver, pancreas, spleen, small intestine, cecum, visceral fat, epididymal fatty tissue and the like which weighed on dissection after four weeks. Furthermore, there was no significant difference in the body length and the intestinal length per unit body weight between both groups.

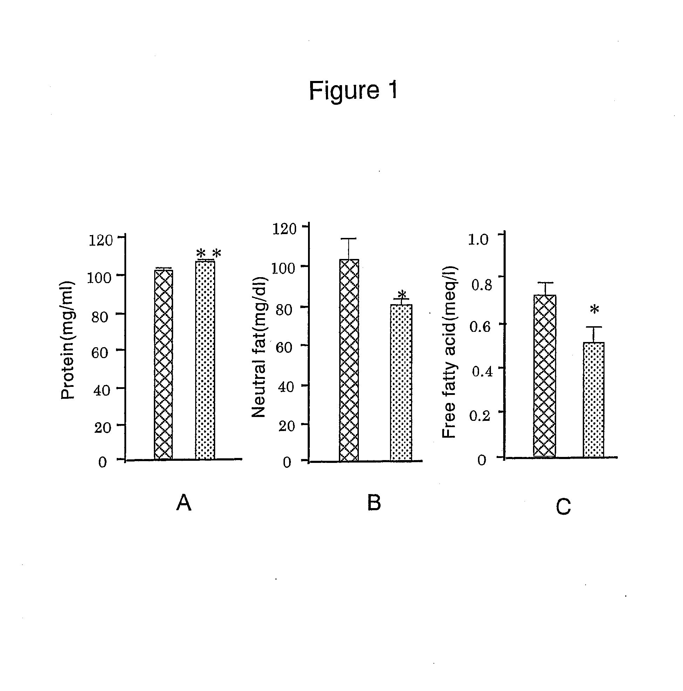

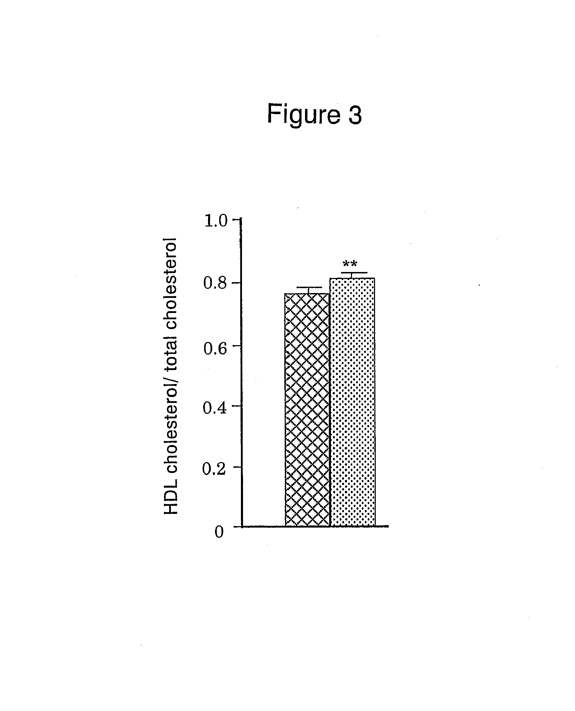

On measuring the blood components, by oral administration of lactoferrin, the blood neutral fat level was 20.8% (P<0.05) (Figure 1B) and the blood free fatty acid level was 27.9% (P<0.05) (Figure 1C), and they were significantly decreased but the total protein concentration in blood was conversely increased (P<0.01) (Figure 1A). Then, on quantitative analysis of the blood cholesterol level, it was recognized that with the lactoferrin group, the total blood cholesterol level had an increasing tendency compared to the control group but became clear that this increase was the result of an increase in HDL cholesterol by 34.5% (P<0.01). The rising in blood HDL cholesterol level is also clear from a significant rising in the ratio of the HDL cholesterol level to the total cholesterol level by 7.1% by the administration of lactopferrin (P<0.05) (Figure 3).

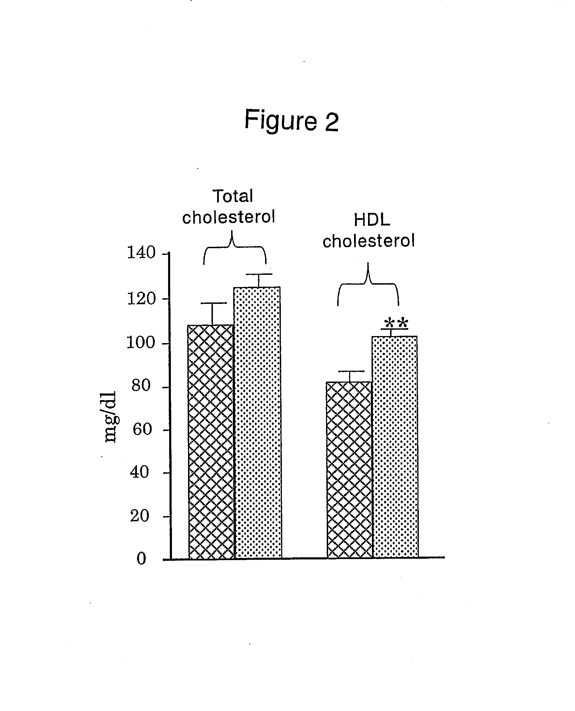

Lactoferrin is a polymer having a molecular weight of a little less than 80,000 Da and has been thought to be hardly absorbed from the digestive tract. However, on oral administration, the action to raise blood protein concentration and to reduce blood neutral fat level and free fatty acid level, furthermore to raise HDL cholesterol were exhibited as shown in this Example (Figures 1, 2 and 3).

If lactoferrin is not absorbed, the highest possibility is that the absorption of lipid in the digestive tract is inhibited. Then, the lipid of the liver where the absorbed dietary fat is stored was measured.

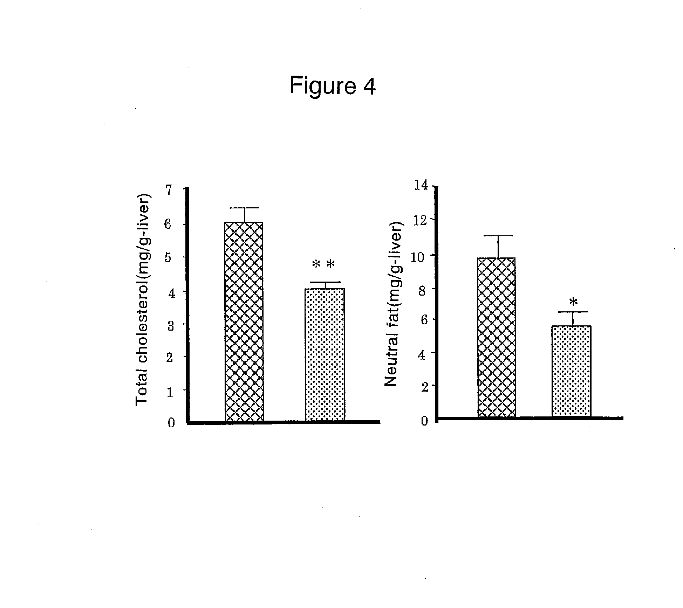

The liver removed from a mouse after four weeks was homogenized with a 2.5 M sucrose-containing phosphate buffer (ph 7.4), and the ground product was added with a mixed solvent of chloroform : methanol (2 : 1) to extract lipid, and cholesterol and neutral fat were measured. By adding 1% of lactoferrin to the standard feed CE-2, the cholesterol content of the liver was reduced by 21.7% (P<0.01) and the neutral fat content was reduced by 41.8% (P<0.05) compared to the control group (Figure 4). In other words, it was assumed that the action of lactoferrin to improve the blood lipid profile could be brought about by the lactoferrin which inhibited the absorption of dietary fat in the digestive tract. It is not known that lactoferrin inhibits the absorption of dietary fat by the digestive tract to improve lipid metabolism and reduces energy intake to exhibit a weight reduction effect as well, and this is a fact which the present inventors have elucidated for the first time.

### Example 2

A male aged 42 took nine enteric coated tablets of lactoferrin (Preparation Example 5) dividedly in three parts after copious drinking (about 500 ml of whisky) (that is, nine tablets a day, equally divided into three parts at rising, before lunch and at bedtime, respectively).

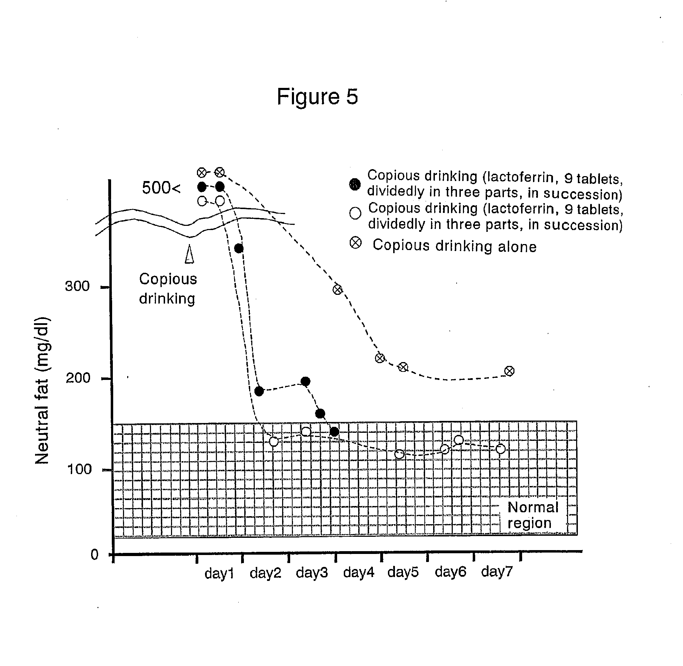

Day 2 after the drinking, the neutral fat level was reduced to the normal region or in its neighborhood. When the enteric coated tablets of lactoferrin were not taken, the neutral fat level was over 200 mg/dl even day 7 after the drinking (Figure 5).

### Example 3

Eight persons having a high total cholesterol level each continuously took nine enteric coated tablets of lactoferrin (Preparation Example of 5) a day, dividedly in three parts.

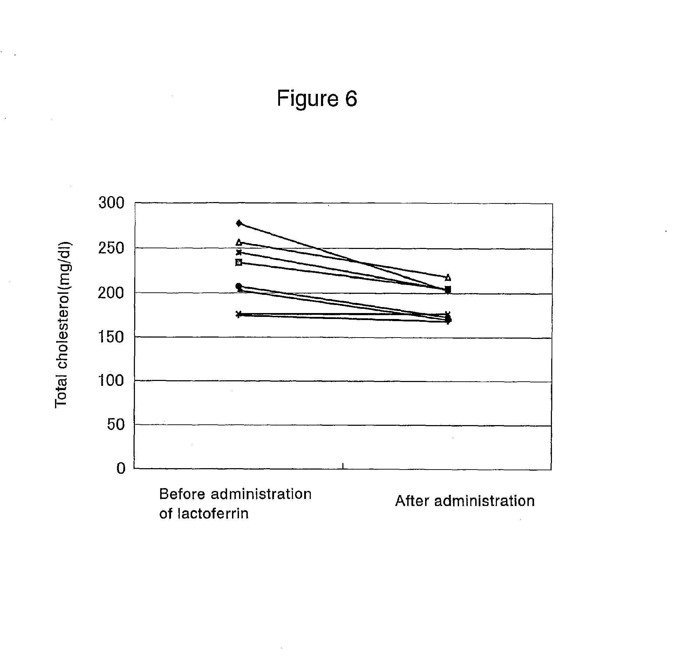

After about one month, with six persons having a total cholesterol level of a little higher than the normal level out of eight persons, the total cholesterol level was reduced (P<0.01 in Student's t-test) but with two persons whose total cholesterol level was in the normal region, the variation of the total cholesterol level was not recognized (Figure 6). From this fact, it can be considered that lactoferrin reduces only the cholesterol unnecessary for a human. Further, the collection of blood was performed at a scheduled time (around 11:00 a.m.).

### Example 4

Twelve able-bodied females continuously took three to nine enteric coated tablets of lactoferrin (Preparation Example 5) a day for about one to two months. During this period, guidance in meals and exercise was not particularly given.

With most persons, the reduction in waist and the reduction in weight were recognized.

### Example 5

A male drinker (a large bottle of beer and one double whisky every day) aged 37 took enteric coated tablets of lactoferrin (Preparation Example 5). At the beginning, the male took nine tablets, dividedly in three parts a day but felt sleepy and could not work, and thus on and after day 2, the male changed to take three tablets at bedtime.

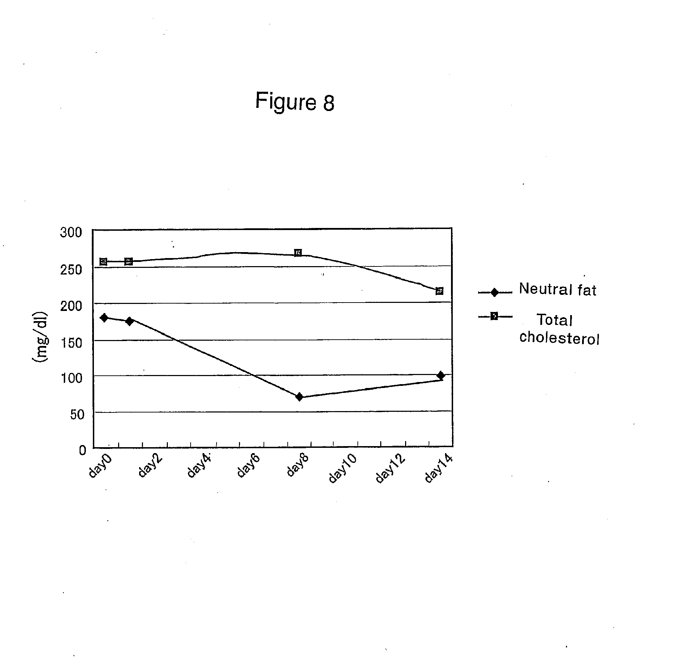

When the neutral fat level was measured day 8, it was significantly reduced. Day 14, the cholesterol level was also reduced (Figure 8). This male had taken mevalotin for a long time but the total cholesterol level was reduced to 210 mg/dl for the first time. During this period, the dietary life was not particularly changed. Further, the collection of blood was performed before lunch.

### Example 6

A female aged 43 having a high neutral fat level and a high total cholesterol level took nine enteric coated tablets of lactoferrin (Preparation Example 5) dividedly in three parts day 1 and day 2, three tablets at bedtime day 3, and six tablets, three tablets at rising and before bedtime, respectively, on and after day 4.

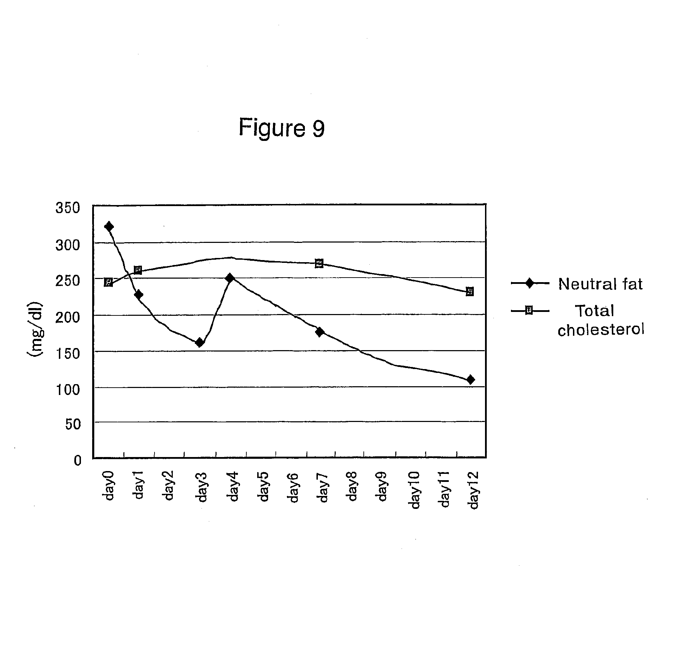

Day 12, the total cholesterol level was reduced to 231 mb/dl (Figure 9). The body weight was also reduced by 2 kg. During this period, the dietary life is not particularly changed. The collection of blood was performed between 10:00 a.m. and 11 a.m.

### Example 7

Due to hyper-triglyceride(TG)-mia, a female aged 41 had taken lipantil for a several months. However, since impaired liver function had appeared, the female stopped taking lipantil and was provided with intravenous injections of Strong Minophargen for six days. The liver function was stabilized but the neutral fat level started rising again.

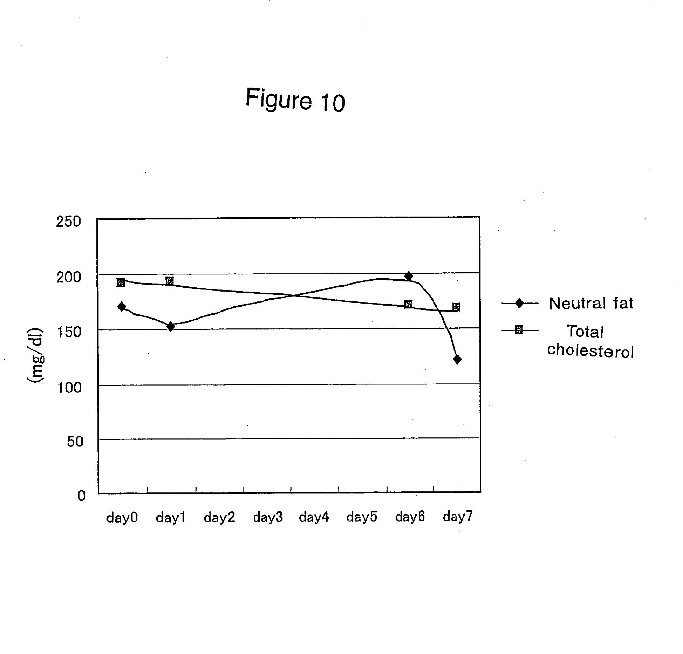

On starting taking lactoferrin enteric coated tablets of lactoferrin (preparation Example 5) (nine tablets, dividedly in three parts), the neutral fat level was 183 mg/dl at the beginning, and reduced to 153 mg/dl the next day. Further, the total cholesterol level which had not been reduced to 200 mg/dl or less without talking lipantil was reduced to 122 mg/dl day 7 (Figure 10). During this period, there was no particular change in the dietary life. The collection of blood was performed between 9:00 a.m. and 10 a.m.

### Example 8

A female aged 65 who was not particularly obese took enteric coated tablets of lactoferrin.

This female only showed a total cholesterol level as high as 250 mg/dl or more and recently had slightly high neutral fat with ages. By taking mevalotin, the total cholesterol level was reduced but due to the side effect of a cramp in the foot or the like, it was impossible to take mevalotin.

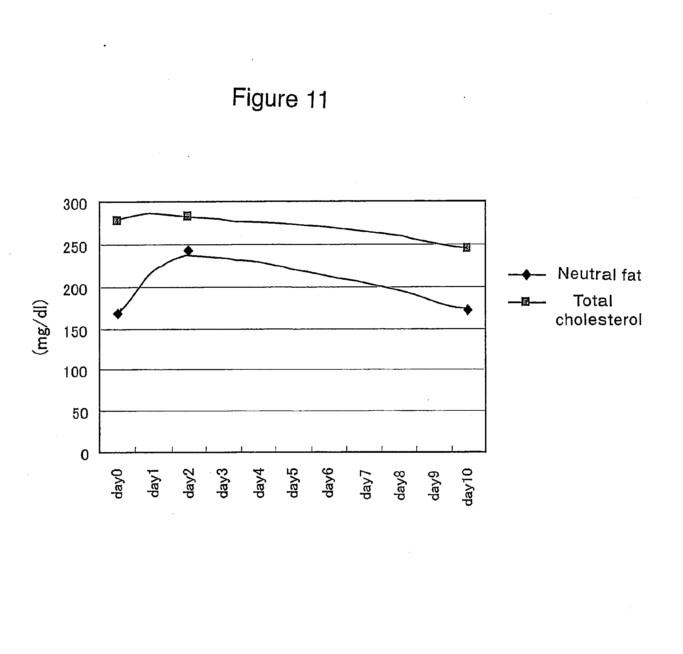

On starting taking enteric coated tablets of lactoferrin (Preparation Example 5) (nine tablets, dividedly in three parts), day 10, the total cholesterol level came to the 240 mg/dl level (Figure 11). During this time, there was no change in the dietary life. The collection of blood was performed between 10:00 a.m. and 11 a.m.

### Example 9

A male drinker aged 42 (about 200 to 300 ml of whisky on an average of once every three to four days) took nine enteric coated tablets of lactoferrin (Preparation Example 5) dividedly in three parts a day.

When the neutral fat level and the total cholesterol level in the collected blood were measured about three times a day, a clear reduction in the total cholesterol level was recognized, and with the neutral fat level, a reducing trendency was observed as the whole although varied depending on days (variation due to meals) (Figure 12). Further, the neutral fat level the next morning after the drinking (shown by ● in Figure 12) was high but the neutral fat level was quickly reduced after discontinuing drinking.

### Example 10

The lactoferrin concentration in blood on oral administration of enteric coated tablets of lactoferrin was measured by the ELISA method using an anti-bovine lactoferrin antibody.

### Measurement of Lactoferrin by ELISA Method

1. Anti-bovine lactoferrin antibody (Goat, anti-bovine LF affinity purified, Bethyl Labor. Co., Ltd.) diluted in 1/500 (2 µg/ml) with a 0.05 M carbonate buffer (pH 9.6) was introduced to a 96-well flat bottom microplate (a product of NUNC Co., Ltd.) in an amount of 100 µl per well and adsorbed at 4°C overnight.
2. The plate was washed three times with a 0.05% Tween 20-phosphate buffer (PBS). As the blocking agent, 300 µl of a 1.3% gelatin-containing PBS was introduced to the plate and incubated at room temperature for 30 minutes.
3. The plate was washed three times with a 0.05% Tween 20-PBS, and a standard or sample diluted with PBS containing 0.05% Tween 20, 0.5M NaCl and 1% bovine serum albumin (BSA) (hereinafter referred to as NB-PBS) was introduced to the plate in an amount of 100 µl/well to effect reaction at 4°C for eight hours.
4. The plate was washed three times with a 0.05% Tween 20-PBS, and an anti-bovine lactoferrin antibody (Rabbit, anti-bovine LF, IgG grade, a product of Yagai Co., Ltd.) diluted in 1/1,000 with NB-PBS was introduced to the plate in an amount of 100 µl/well to effect reaction at 4°C for eight hours.
5. The plate was washed three times with a 0.05% Tween 20-PBS, and peroxidase-labeled anti-rabbit IgG antibody (Goat, anti-rabbit IgG, a product of American Quail International Co., Ltd.) diluted in 1/5,000 with NB-PBS was introduced to the plate in an amount of 100 µl/well to effect reaction at 4°C for eight hours.
6. The plate was washed three times with a 0.05% Tween 20-PBS. Furthermore, 2,2-azino-bis(3-ethyl-benzothiazoline-6-sulfonic acid diammonium salt (1.18 M, a product of Sanko Pure Chemical Co., Ltd.) dissolved in a phosphate buffer was introduced to the plate in an amount of 100 µl/well as a substrate solution to effect reaction at 37°C for one hour.
7. The absorbance at a wavelength of 405 nm was measured by a microplate reader (Sunrise Series, Type Classic, manufactured by Chican Co., Ltd.), and the lactoferrin concentration was calculated from the calibration curve prepared at standards.

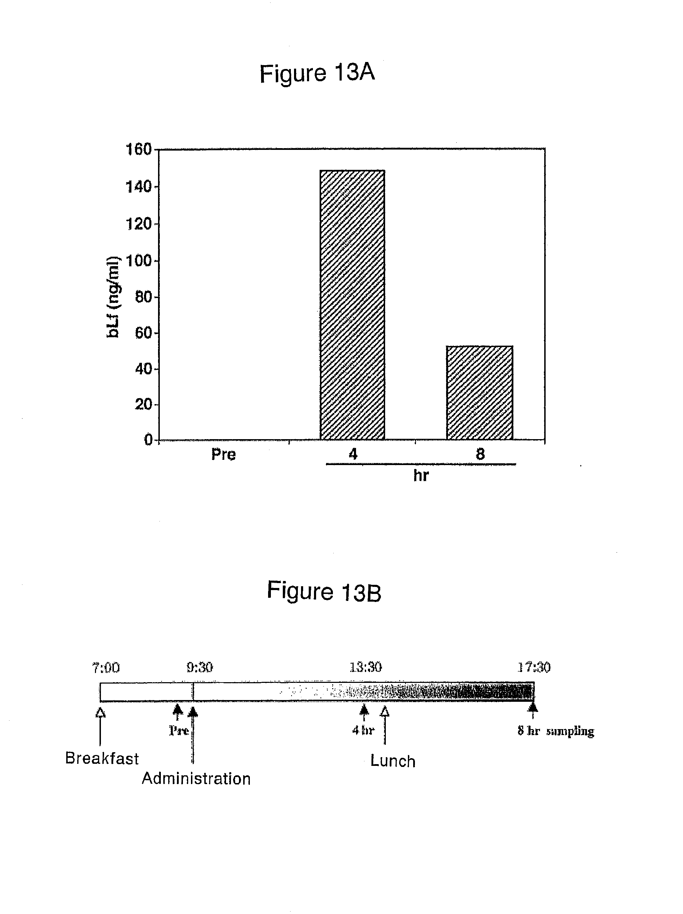

When 18 enteric coated tablets of lactoferrin (900 mg/60 kg=15 mg/kg) (Preparation Example 5) were administered to a male weighing 60 kg, the presence of lactoferrin was confirmed in the blood collected after four hours and eight hours (Figure 13A).

Administration and collection of blood were performed according to the following schedule. Namely, after eating breakfast at 7:00, the blood before the administration of lactoferrin was collected a little before 9:30 (Pre-sampling), and enteric coated tablets of lactoferrin (Preparation Example 5) was administered at 9:30, and then the blood was collected at 13:30 and 17:30 (4 hr-sampling and 8 hr-sampling, respectively) (Figure 13A).

### Example 11

With a group of 11 persons administered with enteric coated tablets of lactoferrin (Preparation Example 5) and a control group of 31 persons, the body temperature at rising and the body temperature one hour after lunch were measured.

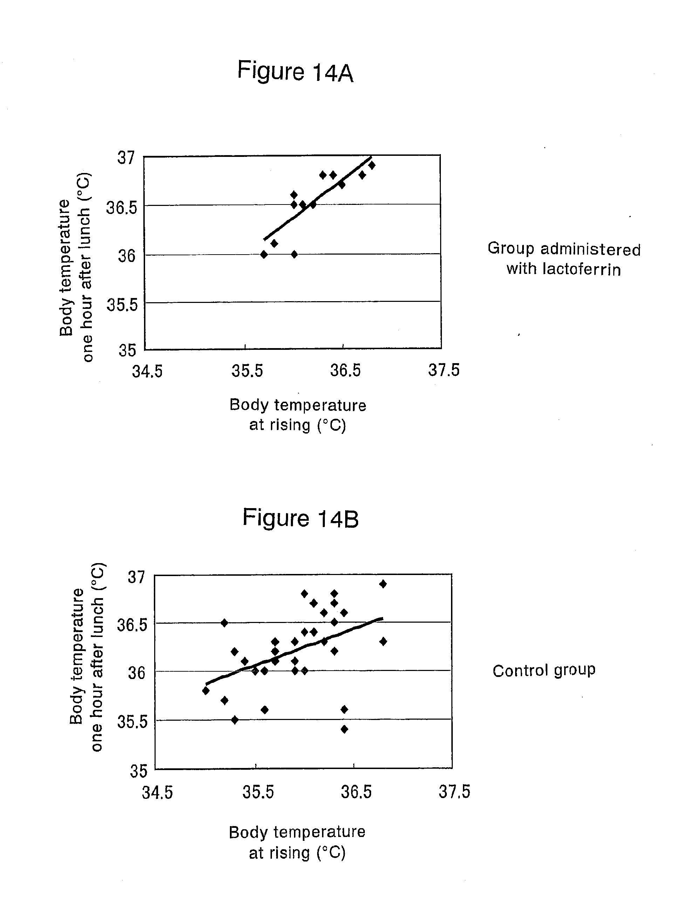

With the group administered with lactoferrin (Figure 14A), the body temperature at rising (P<0.05 in Student's unpaired t-test) and the body temperature one hour after lunch (P<0.01 in Student's unpaired t-test) were both significantly high compared to the control group (Figure 14B). It was considered that with the group administered with lactoferrin, the basal metabolic rate rose compared to the control group.

### EMBODIMENTS

1. A composition for improving lipid metabolism having at least one kind to be selected from the group consisting of a lactoferrin group protein comprising lactoferrin and conalbumin and an enzymatically decomposed product of the lactoferrin group protein comprising peptides corresponding to lactoferricin and lactoferricin of conalbumin as an active ingredient.
2. A composition for treating at least one disease or condition to be selected from the group consisting of hypercholesterolemia, hyper-neutral lipidemia, hyper-low density lipoprotein (LDL) cholesterolemia, hypo-high density lipoprotein (HDL) cholesterolemia, obesity, fatty liver and cholesterol gallstone which has at least one kind to be selected from the group consisting of a lactoferrin group protein comprising lactoferrin and conalbumin and an enzymatically decomposed product of the lactoferrin group protein comprising peptides corresponding to lactoferricin and lactoferricin of conalbumin as an active ingredient.
3. A composition for treating a disease or condition for which the improvement of basal metabolic rate is to be effective which has at least one kind to be selected from the group consisting of a lactoferrin group protein comprising lactoferrin and conalbumin and an enzymatically decomposed product of the lactoferrin group protein comprising peptides corresponding to lactoferricin and lactoferricin of conalbumin as an active ingredient.
4. The composition of any one of embodiments 1 to 3 which is in the form of a dusting powder, a powder, a granule, a tablet or a capsule and can be obtained by the steps comprising mixing the active ingredient with pharmaceutical acceptable additives in the dry state; if desired, subjecting the mixture to strong pressure molding in the dry state and successively forming the molded product into fine particulates or granules of a uniform size; and tableting or encapsulating the mixture, the fine particulates or granules.
5. The composition of any one of embodiments 1 to 4 which is in the form of an enteric coated preparation.
6. The composition of any one of embodiments 1 to 4, wherein tableted granules containing the active component is coated with a film having, as the major component, a base which has resistance to the gastric juice and dissolves in the small intestine.
7. The composition of any one of embodiments 1 to 6 which is for the administration of the active ingredient in an amount of about 0.1 mg to about 50,000 mg, preferably about 0.5 mg to about 10,000 mg, more preferably about 10 mg to about 2,000 mg a day.
8. A method for producing a composition of any one of embodiments 1 to 7, said composition comprising the steps of mixing the active ingredient with pharmaceutically acceptable additives in the dry state; if desired, subjecting the mixture to strong pressure molding in the dry state and successively forming the molded product into fine particulates or granules of a uniform size; and tableting or encapsulating the mixture, the fine particulates or granules, said composition being in the form of a dusting powder, a powder, a granule, a tablet or a capsule.
9. Use of at least one kind to be selected from the group consisting of a lactoferrin group protein comprising lactoferrin and conalbumin and an enzymatically decomposed product of the lactoferrin group protein comprising peptides corresponding to lactoferricin and lactoferricin of conalbumin as an active ingredient in producing a drug for improving lipid metabolism.
10. Use of at least one kind to be selected from the group consisting of a lactoferrin group protein comprising lactoferrin and conalbumin and an enzymatically decomposed product of the lactoferrin group protein comprising peptides corresponding to lactoferricin and lactoferricin of conalbumin as an active ingredient in producing a drug for treating at least one disease or condition to be selected from the group consisting of hypercholesterolemia, hyper-neutral lipidemia, hyper-low density lipoprotein (LDL) cholesterolemia, hypo-high density lipoprotein (HDL) cholesterolemia, obesity, fatty liver and cholesterol gallstone.
11. Use of at least one kind to be selected from the group consisting of a lactoferrin group protein comprising lactoferrin and conalbumin and an enzymatically decomposed product of the lactoferrin group protein comprising peptides corresponding to lactoferrcin and lactoferaricin of conalbumin as an active ingredient in producing a drug for treating a disease or condition for which the improvement of basal metabolic rate is to be effective.
12. The use of any one of embodiments 9 to 11, wherein the drug is in the form of a dusting powder, a powder, a granule, a tablet or a capsule and can be obtained by the steps comprising mixing the active ingredient with pharmaceutically acceptable additives in the dry state; if desired, subjecting the mixture to strong pressure molding in the dry state and successively forming the molded product into fine particulates or granules of a uniform size; and tableting or encapsulating the mixture, the fine particulates or granules.
13. The use of any one of embodiments 9 to 12, wherein the drug is in the form of an enteric coated preparation.
14. The use of any one of embodiments 9 to 12, wherein the drug is obtained by coating tableted granules containing the active ingredient with a film having, as the main component, a base which has resistance to the gastric juice and dissolves in the small intestine.
15. The use of any one of embodiments 9 to 14, wherein the drug is for the administration of the active ingredient in an amount of about 0.1 mg to about 50,000 mg, preferably about 0.5 mg to about 10,000 mg, more preferably about 10 mg to about 2,000 mg a day.
16. The use of any one of embodiments 9 to 15, wherein the drug is in the form of a dusting powder, a powder, a granule, a tablet or a capsule and can be obtained by the steps comprising mixing the active ingredient with pharmaceutical acceptable additives in the dry state; if desired, subjecting the mixture to strong pressure molding in the dry state and successively forming the molded product into fine particulates or granules of a uniform size; and tableting or encapsulating the mixture, the fine particulates or granules.
17. A method of improving lipid metabolism comprising using at least one kind to be selected from the group consisting of a lactoferrin group protein comprising lactoferrin and conalbumin and an enzymatically decomposed product of the lactoferrin group protein comprising peptides corresponding to lactoferricin and lactoferricin of conalbumin as an active ingredient.
18. A method of treating at least one disease or condition to be selected from the group consisting of hypercholesterolemia, hyper-neutral lipidemia, hyper-low density lipoprotein (LDL) cholesterolemia, hypo-high density lipoprotein (HDL) cholesterolemia, obesity, fatty liver and cholesterol gallstone comprising using at least one kind to be selected from the group consisting of a lactoferrin group protein comprising lactoferrin and conalbumin and an enzymatically decomposed product of the lactoferrin group protein comprising peptides corresponding to lactoferricin and lactoferricin of conalbumin as an active ingredient.
19. A method of treating a disease or condition for which the improvement of basal metabolic rate is to be effective comprising using at least one kind to be selected from the group consisting of a lactoferrin group protein comprising lactoferrin and conalbumin and an enzymatically decomposed product of the lactoferrin group protein comprising peptides corresponding to lactoferricin and lactoferricin of conalbumin as an active ingredient.
20. The method of any one of embodiments 17 to 19, wherein the active ingredient is used in the form of a dusting powder, a powder, a granule, a tablet or a capsule which can be obtained by the steps of mixing the active ingredient with pharmaceutically acceptable additives in the dry state; if desired, subjecting the mixture to strong pressure molding in the dry state and successively forming the molded product into fine particulates or granules of a uniform size; and tableting or encapsulating the mixture, the fine particulates or granules.
21. The method of embodiment 20, wherein the active ingredient is in the form of an enteric coated preparation.
22. The method of embodiment 20, wherein the active ingredient is obtained by coating tableted granules containing the active ingredient with a film having, as the main component, a base which has resistance to the gastric juice and dissolves in the small intestine.
23. The method of embodiment 21 comprising administering the active ingredient in an amount of about 0.1 mg to about 50,000 mg, preferably about 0.5 mg to about 10,000 mg, more preferably about 10 mg to about 2,000 mg a day.
24. The method of embodiment 22 comprising administering the active ingredient in an amount of about 0.1 mg to about 50,000 mg, preferably about 0.5 mg to about 10,000 mg, more preferably about 10 mg to about 2,000 mg a day.
25. The method of embodiment 21, wherein the active ingredient is in the form of a dusting powder, a powder, a granule, a tablet or a capsule which can be obtained by the steps comprising mixing the active ingredient with pharmaceutically acceptable additives in the dry state; if desired, subjecting the mixture to strong pressure molding in the dry state and successively forming the molded product into fine particulates or granules of a uniform size; and tableting or encapsulating the mixture, the fine particulates or granules.
26. The method of embodiment 22, wherein the active ingredient is in the form of a dusting powder, a powder, a granule, a tablet or a capsule which can be obtained by the steps comprising mixing the active ingredient with pharmaceutically acceptable additives in the dry state; if desired, subjecting the mixture to strong pressure molding in the dry state and successively forming the molded product into fine particulates or granules of a uniform size; and if desired, tableting or encapsulating the mixture, the fine particulates or granules.

## Claims

1. Food additive or feed additive, comprising a composition containing lactoferrin as the active ingredient.

2. Food additive or feed additive according to claim 1 in the form of a food, a drink or a feed preparation.

3. Food additive or feed additive according to claim 1 or 2, wherein the composition is in the form of a dusting powder, a powder, a granule, a tablet or a capsule.

4. Food additive or feed additive according to any one of claims 1 to 3, wherein the composition is in a dry state.

5. Food additive or feed additive according to any one of claims 1 to 4, wherein the composition is made into enteric coated preparations, optionally wherein the lactoferrin is filled into enteric capsules with a film having, as the major component, a base material which has resistance to the gastric juice and dissolves in the small intestine.

6. A food or feed, comprising the additive according to any one of claims 1 to 5.

7. A food or feed, comprising a composition containing lactoferrin as the active ingredient.

8. A food or feed according to claim 7, wherein the composition is in the form of a dusting powder, a powder, a granule, a tablet or a capsule.

9. A food or feed according to claim 7 or 8, wherein the composition is in a dry state.

10. A food or feed according to any one of claims 7 to 9, wherein the composition is made into enteric coated preparations, optionally wherein the lactoferrin is filled into enteric capsules with a film having, as the major component, a base material which has resistance to the gastric juice and dissolves in the small intestine.

11. Food additive or feed additive according to any one of claims 1 to 5, or food or feed according to claims 6 to 10, for improving the lipid profile in blood.

12. Food additive or feed additive or food or feed according to claim 11, wherein the improvement of the lipid profile is a reduction in blood cholesterol level and/or reduction in blood neutral fat level, optionally a reduction in blood neutral fat level after drinking.

13. Food additive or feed additive according to any one of claims 1 to 5, or food or feed according to claims 6 to 10, for improving basal metabolic rate.

14. Food additive or feed additive according to any one of claims 1 to 5, or food or feed according to claims 6 to 10, for reduction in weight and/or in waist.

15. Use of a composition containing lactoferrin as food or feed additive.

16. Use of lactoferrin for producing a composition that can be added to a food or feed.
